# FASCICULE DE BREVET EUROPEEN

(11) **EP 0 676 207 B1**
(45) Date de publication et mention de la délivrance du brevet: **06.09.2000**
(21) Numéro de dépôt: 95105774.4
(22) Date de dépôt: 13.06.1990
(51) Int. Cl.: A61K 35/80, A61K 31/715

(54) **Agent anticomplémentaire obtenu à partir de fucanes d'algues brunes**
Mittel zur Hemmung des Komplementsystems hergestellt aus Fucanen aus braunen Algen
Anticomplementary agent prepared from fucans of brown seaweed

(30) Priorité: 14.06.1989 FR 8907857
(43) Date de publication de la demande: 11.10.1995
(62) Demande divisionnaire de: 90401636.7
(73) Titulaire: INSTITUT FRANCAIS DE RECHERCHE POUR L'EXPLOITATION DE LA MER (IFREMER), 92138 Issy-les-Moulineaux Cedex (FR)
(72) Inventeur: Colliec, Sylvia, F-44300 Nantes (FR); Bretaudiere, Jacqueline, F-75015 Paris (FR); Durand, Patrick, F-44400 Reze (FR); Fischer, Anne-Marie, F-75013 Paris (FR); Jozefonvicz, Jacqueline, F-60260 Lamorlaye (FR); Kloareg, Bernard, F-29250 Saint-Pol-de-Leon (FR); Vidal, Catherine, F-75020 Paris (FR)
(74) Mandataire: Vialle-Presles, Marie José

(56) Documents cités:
- US-A- 4 098 995
- RESEARCH DISCLOSURE, vol. 289, no. 25, 1 Février 1989 pages 100-101, TETSUO KAWAHARA 'Application of sulfated polysaccharide gel for affinity chromatography.'

## Description

La présente invention est relative à un nouvel agent anticomplémentaire.

Le système du complément est un ensemble de protéines plasmatiques ou membranaires qui jouent un rôle essentiel dans les mécanismes de défense immunitaire. Il intervient dans la défense de l'organisme (destruction des agents infectieux, élimination des complexes immuns) et dans des processus pathologiques de type inflammatoire. Dans le sang il est en outre responsable de l'hémolyse ou lyse des globules rouges.

Les protéines du complément se trouvent sous forme inactivée dans le sérum. Leur activation se fait selon un ordre déterminé selon deux séquences, la voie alterne et la voie classique. L'activation de la voie alterne repose sur un mécanisme non spécifique de reconnaissance de la surface cible (bactérie, virus, parasite, cellule tumorale). L'activation de la voie classique repose sur la reconnaissance spécifique de la cible par un anticorps. Dans la voie alterne, la phase initiale est amorcée par la fixation d'un produit de clivage du C3, le C3b, et la libération du C3a, anaphylatoxine responsable de réactions inflammatoires. Le C3b se fixe ainsi par liaison covalente avec des groupements hydroxyles et aminés de la surface. Une fois lié, il peut fixer le facteur B et après activation de ce dernier par le facteur D, il forme la C3 convertase alterne. Dans la voie classique, la phase initiale est amorcée par l'activation du C1 qui s'effectue principalement au niveau d'un complexe antigène-anticorps. Après activation du C1, les composants C4 et C2 sont à leur tour activés et vont former sur la surface un complexe enzymatique, la C3 convertase classique. L'activation du C4 est analogue à celle du C3 avec libération du C4b et C4a. Le C4b se fixe de manière covalente avec le même type de groupements chimiques que le C3b.

Les inhibiteurs de l'activation du complément peuvent jouer un rôle dans la prévention des phénomènes de rejet des greffes ; leur utilisation a également été suggérée dans le traitement des dialysés rénaux et des convalescents d'infarctus du myocarde. L'activité anticomplémentaire peut également être mise à profit pour inhiber l'activation du complément dans les dispositifs de circulation extra-corporelle

L'héparine, qui est actuellement utilisée comme agent anticoagulant, est également connue pour ses propriétés anticomplémentaires. Elle agit comme inhibiteur de l'activation du complément par son interaction avec les C3 convertases. Il a été montré que cette activité anticomplémentaire de l'héparine est totalement indépendante de son activité antithrombique et qu'elle est liée à la présence sur la fonction amine d'un groupe sulfate ou acétyle, contrairement à l'activité anticoagulante qui n'est pas observée si le substituant de la fonction amine n'est pas un groupe sulfate. Il apparaît également que la présence d'un groupe sulfate sur une des fonctions alcool de l'acide uronique joue un rôle important dans l'activité anticomplémentaire. [KAZATCHINE et al. ; J. Clin. Invest. ; 67, (1981)].

D'autres polysaccharides, tels que par exemple, les dermatanes sulfates, ou le pentosane polysulfate, qui sont également connus pour leurs propriétés anticoagulantes et/ou antithrombotiques ne présentent pas, en revanche, d'activité anticomplémentaire comparable à celle de l'héparine ; KAZATCHINE et al. ont montré, dans la publication citée plus haut, que le dermatane sulfate, dans lequel la fonction amine de la galactosamine est acétylée mais dont les fonctions alcool de l'acide uronique ne sont pas sulfatées, est, sur ce plan, quatorze fois moins actif que l'héparine.

Les fucanes sont des polysaccharides sulfatés, de poids moléculaire moyen élevé (100 à 800 kDa), extraits des thalles d'algues brunes. Ce sont des polymères d'α-1,2-L-fucose-4-sulfate pouvant contenir également du D-xylose, du D-galactose et des acides uroniques. Les acides uroniques des fucanes ne sont pas sulfatés, contrairement à ceux de l'héparine. En outre, les fucanes diffèrent à la fois de l'héparine et du dermatane sulfate, en ce qu'ils ne contiennent pas d'amino-sucres.

Des travaux réalisés sur les fucanes bruts, et utilisant des procédés non agressifs de fractionnement (précipitation fractionnée, filtration sur gel, etc...) qui ne dégradent pas le squelette polysaccharidique des molécules de fucanes ont mis en évidence l'existence de sous-populations naturelles de fucanes, différant les unes des autres par leur poids moléculaire moyen, d'une part, et par leurs propriétés physico-chimiques d'autre part.

Des travaux réalisés sur les fucanes bruts, et utilisant des procédés non agressifs de fractionnement, ont permis de séparer, à partir d'un même extrait brut, diverses sous-populations de fucanes, différant les unes des autres par leur poids moléculaire moyen, d'une part, et par leurs propriétés physico-chimiques d'autre part.

Les Inventeurs ont obtenu des fractions de fucane de faible poids moléculaire en fragmentant par lyse ménagée les chaînes polysaccharidiques du fucane brut d'algues brunes , et en recueillant des fractions de poids moléculaire supérieur à 5 et inférieur à 40 kDa. La lyse ménagée peut être effectuée : par hydrolyse acide résultant de l'action de H₂SO₄ 0,5 à 1N, à une température comprise entre 40° et 50°C, pendant 1 à 4 heures, par radiolyse, en soumettant le fucane à une irradiation, notamment par rayons gamma, ou par hydrolyse enzymatique. Ces fractions, et leur utilisation comme agent anticoagulant et anti-thrombotique, font l'objet de la demande co-pendante EP 0 403 377.

Les Inventeurs ont constaté que, de façon surprenante, les fucanes, bien qu'ils ne contiennent (contrairement à l'héparine) ni amino-sucres, ni acides uroniques O-sulfatés, ont la propriété d'inhiber l'activation du complément. Les fractions d'un poids moléculaire compris entre 5 et 100 kDa, obtenues à partir des fucanes, possèdent une activité anticomplémentaire égale ou supérieure à celle des fucanes non fractionnés.

La présente invention a pour objet l'utilisation de fucanes extraits de Phéophycées et/ou de leurs fractions pour l'obtention d'un agent inhibiteur de l'activation du complément.

Selon un mode de mise en oeuvre préféré de la présente invention, on utilise une fraction de poids moléculaire supérieur à 50 kDa, obtenue à partir d'un fucane extrait de Phéophycées.

Selon un autre mode de mise en oeuvre préféré de la présente invention, on utilise une fraction de poids moléculaire moyen compris entre 5 et 50 kDa, obtenue à partir d'un fucane extrait de Phéophycées.

Avantageusement, on utilise une fraction de polysaccharides sulfatés obtenue par lyse ménagée de fucane brut extrait de phéophycées, conformément à la Demande EP 0 403 377.

La présente invention sera mieux comprise à l'aide du complément de description qui va suivre, et qui se réfère à des exemples de démonstration de l'activité anticomplémentaire de fucanes extraits de différentes algues brunes, et de leur fractions.

### EXEMPLE 1 : DETERMINATION DE L'ACTIVITE ANTICOMPLEMENTAIRE GLOBALE DES FUCANES ET DE LEURS FRACTIONS.

L'activité anticomplémentaire est déterminée par dosage du CH50 (complément hémolytique 50), en présence ou en l'absence de fucanes. Le dosage du CH50 permet d'apprécier l'activité fonctionnelle globale de la voie classique du système du complément du sérum humain. Ce test consiste à déterminer la plus petite quantité de sérum humain fraîchement recueilli capable d'entraîner la lyse de 50 % d'un nombre donné de globules rouges de mouton sensibilisés de façon optimale par des anticorps de lapin antiglobules rouges de mouton (Es = Erythrocytes sensibilisés).

Les protéines de la voie classique du système du complément reconnaissent ces globules rouges comme un élément étranger et s'activent par clivages successifs pour réagir sur ces globules rouges, ce qui entraîne la lyse de ceux-ci. Il est possible de déterminer les propriétés d'inhibition d'un polymère tel que l'héparine ou le fucane en dosant le CH50 en présence du polymère. La capacité d'inhibition est donnée par la concentration en mg de polymère par ml de sérum humain dilué au 1/4 capable d'inhiber 50% de la lyse des cellules, en établissant une courbe dose/réponse de la lyse des cellules en fonction de la concentration en polymère. Plus celle-ci est faible, plus l'activité anticomplémentaire du polymère est importante. Pour mesurer l'activité anticomplémentaire des fucanes, l'extrait acide (fucane brut) ainsi que les fractions de différents poids moléculaires obtenues par hydrolyse acide dudit fucane brut, décrites aux Exemples 1 et 2 de la Demande EP 0 403 377 ont été utilisés. Les conditions expérimentales sont les suivantes :

50 µl de SHN (sérum humain normal) pur sont mélangés avec 50 µl de fucane à différentes concentrations(0,2 à 0,5 mg/ml) ; après une incubation de 30 minutes à 37°C, 4900 µl de tampon VBS++ sont ajoutés. On obtient la solution A.

D'autre part, 0,3 à 0,8 ml de tampon VBS++ sont mélangés à 0 à 0,5 ml de la solution A, pour obtenir un volume total de 0,8 ml ; 0,2 ml d'érythrocytes sensibilisés sont ajoutés au mélange ; après une incubation de 45 minutes à 37 °C, le mélange est centrifugé pendant 10 min à 1300 g et à 4°C, et l'on procède à la lecture de la DO à 414 nm.

| Composition du tampon VBS⁺⁺ : | |
|---|---|
| Nacl | 42,5 g |
| Véronal (diethyl-barbital de sodium) | 1,875 g |
| Acide diéthyl-barbiturique | 2,85 g |
| H₂O distillée qsp | 1000 ml |

Le pH est ajusté à 7.4 ; 20 ml de cette solution sont mélangés à 80 ml d'eau distillée : on ajoute 0,5 ml de CaCl₂ 0,03 M et 0,5 ml de MgCl₂ 0,10 M.

Les résultats obtenus sont reportés dans le tableau I (2è colonne). Les fractions de fucanes sont 3 à 100 fois plus actives que l'héparine H108 analysée dans les mêmes conditions. Cette activité est indépendante de l'activité anticoagulante.

**Tableau I**

| Fractions | DI₅₀ (CH50) (mg/ml) | DI₅₀ C3a (mg/ml) | DI₅₀ C4a (mg/ml) |
|---|---|---|---|
| EA An | 0,035 - 0,16 | nd | 0,14 |
| A₁ An | 0,26 | 0,070 | nd |
| A₂ An | 1 - 1,37 | 0,10 | 0,11 |
| A₃ An | 0,20 | 0,175 | 0,024 |
| A₁ Pc | 0,036 | nd | nd |
| A₂ Pc | 0,60 | nd | nd |
| A₂ Fv | 1,95 | nd | nd |
| A₂ Up | 1,26 | nd | nd |
| Héparine | 4 | 0,15 | 0,05 |

Il est ainsi montré que le fucane brut ainsi que des fractions de poids moléculaire moyen compris entre 5 et 100 kDa, inhibent totalement l'activation du complément et possèdent une activité anticomplémentaire supérieure à celle de l'héparine. En effet, alors qu'il faut dans le cas de l'héparine, 4 mg/ml de polymère pour inhiber 50 % de la lyse des cellules, il ne faut que 0,035 à 1,95 mg/ml (suivant le poids moléculaire) de fucanes bruts ou fractionnés, pour obtenir le même effet. Il a été ainsi observé que des fractions de poids moléculaire moyen 18 000 Da, préparées comme décrit à l'exemple 1 de la demande EP 0 403 377, inhibent 50% de la lyse des cellules à une concentration de 1,3 mg/ml et que des fractions de poids moléculaire moyen compris entre 5 et 10 kDa inhibent 50% de la lyse des cellules à une concentration de 0,20 mg/ml.

### EXEMPLE 2 : MESURE DU C3a ET DU C4a PAR RADIOIMMUNOESSAI

La concentration des protéines C3a et C4a libérées en phase fluide au cours de l'activation est mesuré dans le surnageant par dosage radioimmunoessai.

L'essai s'effectue de la façon suivante à l'aide du kit de radioimmunoessai C3a-desarg 125I (commercialisé par Amersham)

50 µl de SHN pur activé par le Séphadex, sont mélangés avec 50 µl de tampon VBS++, et 100 µl de fucane à des concentrations variables (0,1 à 5 mg/ml) ; le mélange est incubé 30 minutes à 37 °C puis centrifugé pendant 5 mn à 1700g. On prélève 160 µl de surnageant, auxquels on ajoute 10 µl d'EDTA (8,66.10⁻² M), ( qui bloque l'activation du complément), puis 170 µl d'agent précipitant (fourni avec le kit de dosage) ; après une incubation de 5 minutes à température ambiante, le mélange est centrifugé pendant 2 minutes à 10 000 g, et le surnageant est dilué (1/4 à 1/512), avant de procéder au dosage RIA

L'activité de l'échantillon (DI50) est exprimée comme la quantité de fucane nécessaire pour inhiber 50% de la quantité de C3a normalement généré dans 1 ml de SHN dilué au 1/4. cette dose inhibitrice est exprimée en mg de fucane/ml de SHN dilué au 1/4. Les résultats sont reportés dans le tableau I (colonne 3).

Une solution de 0,21 mg/ml de A₂(An) dans du SHN dilué au 1/4 permet de diminuer l'activation du C3 en dessous de l'activation spontanée de SHN.

En outre, 0,5 mg de la même fraction suffisent à inhiber totalement l'activation du C3 contenu dans 1 ml de SHN dilué au 1/4 et soumis à un activateur (Sephadex, qui entraîne, à la concentration de 15 mg/ml une activation totale du complément) mis en quantité supérieure à celle nécessaire à une activité totale du complément.

Les résultats relatifs au C4a sont également reportés dans le tableau I (colonne 4).

## Revendications

1. Utilisation de fucanes extraits de Phéophycées et/ou de leurs fractions, pour l'obtention d'un médicament inhibiteur de l'activation du complément.

2. Utilisation selon la Revendication 1, caractérisée en ce que l'on met en oeuvre une fraction de poids moléculaire supérieur à 50 kDa obtenue à partir d'un fucane extrait de Phéophycées.

3. Utilisation selon la Revendication 1, caractérisée en ce que l'on met en oeuvre une fraction de poids moléculaire moyen compris entre 5 et 50 kDa, obtenue à partir d'un fucane extrait de Phéophycées.

4. Utilisation selon la Revendication 3, caractérisée en ce que l'on met en oeuvre une fraction de poids moléculaire moyen compris entre 5 et 40 kDa, obtenue par lyse ménagée à partir d'un fucane extrait de Phéophycées.

## Patentansprüche

1. Verwendung von aus Pheophyceen extrahierten Fucanen und/ oder deren Fraktionen zum Erhalt eines Medikaments zur Hemmung der Aktivierung des Komplementsystems.

2. Vewendung nach Anspruch 1, dadurch **gekennzeichnet,** daß man eine aus einem aus Pheophyceen extrahierten Fucan erhaltene Fraktion mit einem Molekulargewicht oberhalb von 50 kDa einsetzt.

3. Verwendung nach Anspruch 1, dadurch **gekennzeichnet,** daß man eine aus einem aus Pheophyceen extrahierten Fucan erhaltene Fraktion mit einem mittleren Molekulargewicht zwischen 5 und 50 kDa einsetzt.

4. Verwendung nach Anspruch 3, dadurch **gekennzeichnet,** daß man eine durch schonende Lyse eines aus Pheophyceen extrahierten Fucans erhaltene Fraktion mit einem mittleren Molekulargewicht zwischen 5 und 40 kDa einsetzt.

## Claims

1. Use of fucans extracted from Pheophyceae and/or of fractions thereof in order to obtain a drug which inhibits complement activation.

2. Use according to Claim 1, characterised in that a fraction is used having a molecular weight greater than 50 kDa, which is obtained from a fucan extracted from Pheophyceae.

3. Use according to Claim 1, characterised in that a fraction is used having an average molecular weight of between 5 and 50 kDa, which is obtained from a fucan extracted from Pheophyceae.

4. Use according to Claim 3, characterised in that a fraction is used having an average molecular weight of between 5 and 40 kDa, which is obtained by mild lysis from a fucan extracted from Pheophyceae.
